# EUROPEAN PATENT APPLICATION

(11) **EP 4 806 998 A1**
(43) Date of publication of application: **16.09.2026**
(21) Application number: 25163780.7
(22) Date of filing: 14.03.2025
(51) Int. Cl.: C12N 15/10, C12Q 1/6806, C40B 40/06

(54) **DATA STORAGE IN DNA**

(71) Applicant: CUBO Biotechnologies AB, 116 31 Stockholm (SE)
(72) Inventor: Ran, Caroline, 128 46 Bagarmossen (SE); Perez, Cynthia, 11631 Stockholm (SE)
(74) Representative: Brann AB

(57) **Abstract**

The present document is directed to storage of data using nucleic acids. According to the present document, existing nucleic acids are used for preparing a library of fragmentised nucleic acid, into which data can be encoded. The fragments of nucleic acid with encoded data are used for storing data by assembling longer fragments of nucleic acid sequences having a sequence corresponding to the data code to be stored.

## Description

### TECHNICAL FIELD

The present document is in the field of storing data using nucleic acid. More particularly, it relates to a method for preparing a nucleic acid library that may e.g., be used for data storage, the library as such and use of the existing nucleic acid for the preparation of a nucleic acid library for data storage.

### BACKGROUND

Due to exponentially increasing needs of data storage and the limitations of current technologies for data storage, DNA data storage has been proposed as a future sustainable solution. The first demonstration of data storage in DNA was done in an art project by the artist Joe Davis in 1996. Davis translated the Algiz rune (the "z"-rune" of the Elder Futhark alphabet) to a binary code of zeroes and ones, then converted it into a DNA sequence of nucleotides that was put into living *E. coli* bacteria.

Since this first example, the feasibility of storing data in DNA has been demonstrated also by others. DNA constitutes an optimal medium to store data due to its high information density, stability, and energy efficiency. Importantly, DNA is the medium developed by nature to store the data of all living organisms and it has been optimized for millions of years to efficiently store and transmit all information contained in organisms. DNA can preserve information even when kept in suboptimal conditions for molecular preservation. For example, the molecular structure and sequence of DNA can be read from samples dated 52,000 years old (Sandoval-Velasco 2024).

The stability of the DNA molecule leads to unprecedented advantages for the purpose of molecular data-storage as the end-product will require no maintenance or other types of digital device to remain functional. A gram of DNA can store up to 215 petabytes of data, implying that the world's data could fit in a small volume and be archived with negligeable energy consumption and maintenance for millennia.

One of the challenges in synthesizing DNA, has been to efficiently generate fragments longer than 3000 base pairs (bp). This is due to methodological challenges that range from the chemistry employed in the DNA synthesis process to the molecular biology methods utilized. DNA synthesis is currently not efficient enough to allow for large scale synthesis of de novo DNA sequence. Current limitations on the market are DNA fragments of around 5000 bp, although larger fragments may be obtained, the synthesis of longer fragments is not standard and does not allow for industrial scaling.

Thus, to make commercial DNA data storage possible, efficient, and affordable DNA synthesis has to be enabled as well as to understand how to synthesize and generate the longer DNA fragments needed for such storage.

DNA synthesis is the process of constructing a new strand of DNA nucleotide by nucleotide, controlling the DNA sequence and length. The state-of-the-art DNA synthesis has advanced significantly over the past decades. Specifically, the use of solid-phase chemical synthesis, the most common method for synthesizing short oligonucleotides, allows the production of high-quality and high-yield short DNA sequences. This method is based on phosphoramidite chemistry and synthesizes DNA by sequential addition of nucleotides to a growing DNA chain attached to a solid support. Phosphoramidite chemistry DNA synthesis has been automated and integrated into high-throughput platforms by several companies that are now leading the in the field of DNA synthesis.

Another approach that has been used for DNA synthesis is enzymatic DNA synthesis. Enzymatic DNA synthesis comprises enzymatic synthesis of DNA strands as well as enzymatic ligations of preexisting strands e.g., Gibson or Golden Gate assemblies. There are many enzymes enabling to build DNA sequences from a preexisting template sequence (DNA polymerases). To generate novel strands of DNA, Terminal Deoxynucleotidyl Transferase (TdT) mediated synthesis is a future possibility, but still under development. TdT enzymes add nucleotides to the 3' ends of single-stranded DNA without a template and is commonly used for labeling pre-existing DNA by incorporating modified nucleotides, adding overhangs or tails or generate random DNA sequences. In order to use TdT mediated synthesis to generate DNA molecules with programmable sequences the incorporation of new nucleotides to the 3' end has to be controlled, which is challenging with present day methodology.

Despite these advances, there are still limitations to DNA synthesis technology. One of the main challenges is the cost and low scalability of the present approaches, which are currently prohibiting their use in DNA data storage, but also in other applications in medicine and synthetic biology.

Native DNA has been used in the development of new approaches for data storage in DNA. For example, US11538554 discloses the extraction of genomic DNA from *E.coli* bacteria which is then nicked by enzymatic activity. The presence or absence of a nick at each nicking site within the genetic code is encoded as binary code (0 or 1). In this way the DNA can be used for data storage without considering the sequence of nucleotides as the digital data is encoded in DNA by these engineered modifications.

WO 2016/059610 describes a method of encoding digital data into an existing genetic sequence. The approach starts by converting digital data to a nucleotide sequence, which is then divided in chunks of four nucleotides, which can be any of the 256 possible combinations of ATC and G. These 256 combinations are in a second stage mapped to the genome of an organism (here the genetic code of *E.coli* was used) marking the start and end position for each sequence of 4 nucleotides. By reading the genetic code of *E.coli* and referring to the position of these 4 nucleotide sequence bouts it is then possible to retrieve the original data.

WO 2021/231920 describes a solution for encoding data in DNA and thereafter reading the data in a nanopore device by usage of a template DNA strand which is hybridized to different addresses and blockage oligos. These hybridizing oligos are used to manage how the DNA strand is read in the nanopore. Double stranded DNA gets caught in the nanopore reading device, and when the oligos are detached from the DNA template the read continues. If the oligos are designed intelligently, they can be used to manage frameshift encoding in the DNA sequence. This allows different codons to be defined within the same sequence of template DNA, by simply shifting the reading frame by usage of multiple address tags.

US2020063119 discloses *in vitro* DNA manipulation with nucleotide precision which enables the encoding of information in a DNA sequence using sequence modifying enzymes. This uses natural occurring DNA (genomic, bacterial or plasmids isolated from an organism or synthesized) as storage medium for the DNA storage. A specific sequence in the storage medium DNA is targeted by a multitude of target DNA or DNA sequences coupled to base modifying enzymes which enable precision editing of nucleotides in the target nucleotides. The information encoded in the DNA can later be read by detecting the edited bases.

Another challenge of de novo DNA synthesis lies within complex fragment assemblies. Complex fragment assembly involves creating libraries of oligonucleotides which can then be assembled together in any combination to create longer fragments of desired sequence. The method comes with the advantages of not having to generate base by base polymers sequences *de novo.* On the other hand, fragment assemblies such as we know them today rely on the inclusion of overhangs that can direct the ligations, tags, and specific sites for directed primers and probes. The inclusion of these sequences implies that the sequence becomes longer and more complex, and DNA created in this way cannot be used for other purposes i.e., synthetic biology or pharmaceutical applications unless the tag-sequences are first removed. Catalog technologies have proposed a system called Cartesian Product of Components (CPC) where they use predefined sets of components that can be ligated to each other in a systematic scheme as building blocks to create longer fragments. However, this technology, as well as other similar solutions based on the generation of oligonucleotide libraries to minimize synthesis of long strands, depend on classic phosphoramidite chemistry to synthesize the short oligonucleotides that are incorporated in the libraries, and do not solve the challenge for the efficient generation of long DNA fragments.

Traditionally other organisms like yeast have been used to generate longer DNA fragments. One method used to generate long sequences of synthetic DNA is Polymerase Cycling Assembly (PCA). PCA employs overlapping oligonucleotides that collectively represent an entire genome. These oligonucleotides are designed with complementary regions to facilitate hybridization. Other methods of relevance are Gibson Assembly, which utilizes a set of DNA fragments with overlapping ends, T5 exonuclease, Phusion DNA polymerase, and Taq DNA ligase. Transformation-Associated Recombination (TAR) is another approach which employs yeast artificial chromosomes (YACs) where DNA fragments are introduced into yeast cells, where they recombine into larger DNA constructs. This method has been used to assemble genomes up to 600 kilobases, such as the *Mycoplasma genitalium* genome. Finally, microchip-based gene synthesis utilizes microchip technologies to synthesize DNA oligonucleotides in a parallel fashion, assembling larger DNA constructs base by base. Although these methods have provided means to increase the lengths of DNA polymers, better approaches are needed that can scale and at the same time diminish the costs for generating >5000bp long fragments.

In living cells, genome stability is maintained by histones. The entire tree of life is populated by histones. Histones are needed and preserved through evolution in many organisms. In the mammalian genome, 4 histone proteins take care of organizing the genome, by wrapping around 147bp DNA fragments. The 4 main histone proteins are H1, H2, H3, H4. Histone reconstitution assays have been traditionally used for *in-vitro* studies of histone molecules and chromatin dynamics. Condensin is a highly conserved protein complex present across evolution, from bacteria to eukaryotes. It plays a critical role in the structural organization, compaction, and segregation of chromosomes during cell division. The emergence of two distinct condensin complexes in eukaryotes is hypothesized to be an evolutionary adaptation to the increasing complexity of chromosomal architecture required by larger genomes and multicellular life. In prokaryotes, bacterial genomes, which range in size from approximately 1 to 10 megabase pairs (Mb), also utilize condensin-like proteins for chromosome organization. For example, condensin-like complexes such as the Smc-ScpAB system play an essential role in maintaining chromosome structure and contribute to genome stability during replication in organisms with genomes within this size range.

WO2001081370A2 describes the usage of histones to stabilize DNA sequences for subsequent usage in molecular applications (transfections and nuclear localization). In this document, short peptides derived from histones, not entire histone proteins or protein complexes are used for stabilizing DNA sequences.

US11753744B2 describes compositions and methods for DNA barcoding of designer mononucleosome and chromatin array libraries, enabling the profiling of chromatin-associated proteins. These include chromatin readers, writers, erasers, and modulators, which regulate gene expression by interacting with chromatin. By incorporating unique DNA barcodes into synthetic nucleosomes, the method allows high-throughput identification and functional characterization of these chromatin regulators. This technology can be used for drug discovery, epigenetic research, and understanding chromatin dynamics in various biological processes with the purpose of gaining knowledge of nucleosomes with respect to variations and modifications occurring *in vivo.*

Therefore, even if several technologies have been proposed to store data using DNA and to generate the longer DNA fragments necessary for large scale DNA-based data storage, the present methods are still not affordable or efficient enough to allow for large scale and/or industrial synthesis of *de novo* DNA for data storage and other applications. Further, once longer fragments of DNA are produced, problems with secondary structure of the DNA chains arise which have not yet been solved.

An objective of the present invention is thus to overcome or at least mitigate one or more of the problems described herein.

### SUMMARY

One aim of the present document is thus the exploration of new technologies for DNA synthesis that relies in concepts of molecular biology applied for generating new strands of nucleic acid and lengthening the nucleic acid chain beyond the 5000 bp mark. Also, the present document is directed to how the nucleic acid sequences can be stably and efficiently extended in length while preventing secondary structure formation.

The present document is thus directed to a method for producing a nucleic acid library for data storage, said method comprising the steps of:
i) providing a nucleic acid;
ii) fragmenting the nucleic acid of step i) to provide oligonucleotide fragments of nucleic acid;
iii) isolating the oligonucleotide fragments of nucleic acid obtained in step ii) to provide individual oligonucleotide fragments of nucleic acid;
iv) optionally sequencing the individual oligonucleotide fragments of nucleic acid obtained in step iii);
thereby providing a library of individual oligonucleotide fragments for data storage.

The present document is also directed to a nucleic acid library for data storage, wherein the nucleic acid library for data storage is obtained or obtainable by the method for producing a nucleic acid library for data storage.

The present document is also directed to a method for storing data, said method comprising storing data using a nucleic acid library for data storage as defined herein.

The present document is also directed to the use of nucleic acid, such as native nucleic acid, for the preparation of a nucleic acid library for data storage.

Other features and advantages of the invention will be apparent from the following detailed description, drawings, examples, and from the claims.

### DEFINITIONS

The singular forms "a", "an" and "the" include plural references unless the context clearly dictates otherwise.

### BRIEF DESCRIPTION OF DRAWINGS

Figure 1 shows DNA extracted from corn after purification by centrifugation and cleaning with AMPure beads. While some protein residues remain, the DNA can be used for further downstream applications or further cleaning if needed. 149.03 ng/µL, 260/280nm = 1.94, 260/230nm = 1.59.

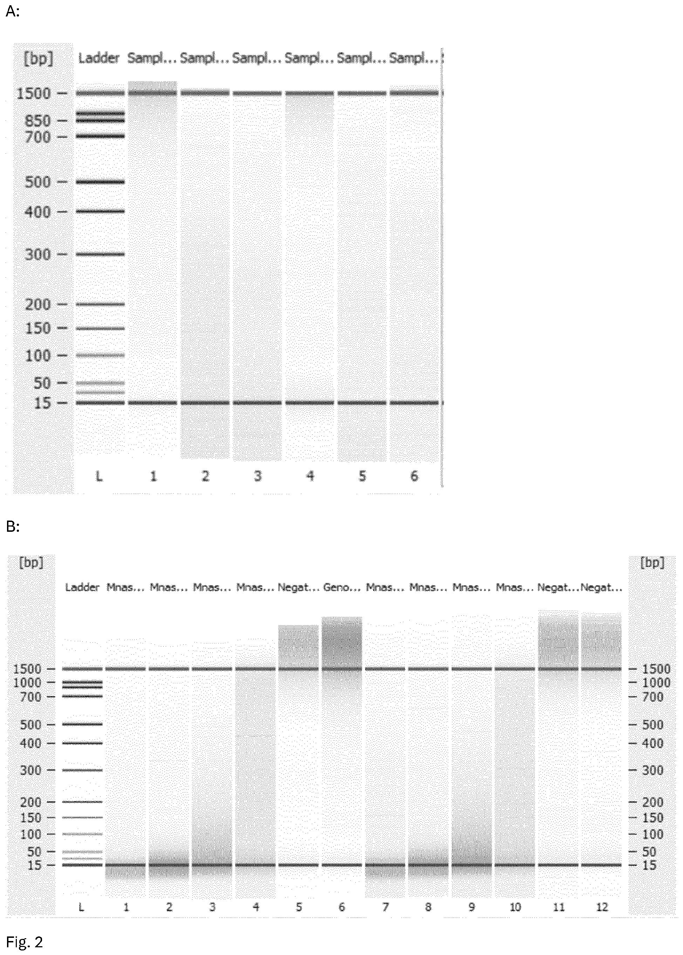

Figure 2 shows in A: Agilent 2100 Bioanalyzer analysis of unfragmented and enzymatically fragmented DNA using an Agilent DNA 1000 kit. Lane L: ladder, lanes 1 and 4: unfragmented DNA; Lanes 2 and 5: DNA digested for 2 min using Micrococcal Nuclease, lanes 3 and 6: DNA digested for 10 min. B: Agilent 2100 Bioanalyzer analysis of DNA enzymatically fragmented using an Agilent DNA 1000 kit. DNA was incubated with different concentrations of micrococcal nuclease for 2 min at 37°C. Lane L: ladder, 1: Nuclease dilution 1:8; 2: Nuclease dilution 1:16; 3: Nuclease dilution 1:32; 4: Nuclease dilution 1:64; 5: negative control (no nuclease); 6: unfragmented genomic DNA. Lanes 7 - 12: technical replicates.
Figure 3 shows Agilent 2100 Bioanalyzer electropherograms of DNA enzymatically fragmented, panels A and B shows DNA fragmented using micrococcal nuclease diluted 1:8 (A) and 1:16 (B) resulting in different sized fragments.
Figure 4 shows a 2% Agarose gel with DNA samples digested by Micrococcal Nuclease. The gel is loaded with GeneRuler 1 kb Plus DNA Ladder (lane 2), TriDye^{™} Ultra Low Range DNA Ladder (lane 4) and DNA digested by Micrococcal Nuclease diluted 1:16 (lane 6). Remaining lanes on the gel are empty.
Figure 5 illustrates the separation of fragments according to the principle of serial dilutions.

### DETAILED DESCRIPTION

The present document presents a new methodology for digital data storage using nucleic acid. By fragmentation of existing (such as native) nucleic acid, a random and elastic nucleic acid library is generated. This library doesn't require complex chemistries or the manipulation of enzymes for its utilization in nucleic data storage and other synthetic biology applications, as it utilizes already existing nucleic acid extracted from organisms which can be cheaply cultured in the lab, like *E. coli,* bacteria or any other available source of nucleic acid such as disposed waste food. Compared to other solutions, the present solution is unique in the usage of existing nucleic acid. Further, the present solution allows the generation of nucleic acid sequences of the desired length and sequence by assembly of shorter oligonucleotide fragments into computational strands, which can be done on demand. Recycling existing strands of nucleotides and use in the synthesis of new nucleic acid greatly decreases the costs of nucleic acid synthesis.

In accordance with the present document, by developing a more efficient nucleic acid synthesis technology in addition to a way to elongating oligonucleotide fragments to several thousand bp long fragments (such as beyond the 5000 bp current standard it is possible to advance a range of industries who depend on synthetic biology, including pharmaceuticals, agriculture, material science and bioengineering. Enabling the creation of longer, more accurate nucleic acid sequences, could for example facilitate advancements in gene therapy, allowing for the development of more complex genetic treatments but also our understanding of genomes. Additionally, this breakthrough could open new possibilities for designing synthetic organisms, programable medicines, and creating sustainable alternatives to traditional chemical processes. Overcoming the present limitations in length of synthetic nucleic acid would not only push the boundaries of current state of the art on nucleic acid synthesis technologies but also establish a foundation for innovations that were previously considered not feasible.

The present document is directed to a method for producing a nucleic acid library for data storage using existing nucleic acid instead of *de novo* synthesising nucleic acid when preparing the nucleic acid library. The method comprises the steps of:
i) providing a nucleic acid;
ii) fragmenting the nucleic acid of step i) to provide oligonucleotide fragments of nucleic acid;
iii) isolating the fragments of nucleic acid obtained in step ii) to provide individual oligonucleotide fragments of nucleic acid;
iv) optionally sequencing the individual oligonucleotide fragments of nucleic acid obtained in step iii);
thereby providing a library of individual oligonucleotide fragments of nucleic acid for data storage.

The oligonucleotide fragments of nucleic acid produced in the method are typically from about 4 bp to about 30 bp long, such as from about 4 bp to about 25 bp, such as from about 4 bp to about 20 bp, such as from about 4 bp to about 15 bp or from about 4 bp to about 10 bp, such as from about 5 bp to about 15 bp or about 10 bp. The advantages with these sizes will be explained further in the below.

The oligonucleotide fragments of nucleic acid are used as building blocks and form a library of nucleic acid sequences that can be used for any secondary purpose, by themselves or in different combinations. The oligonucleotide fragments of different lengths build a vast library of "n" nucleic acid sequences which can be elastic in fragment sizes and random.

Digital data is encoded into a sequence of oligonucleotides by assigning each binary number (or combination of binary numbers) with a nucleic acid and then producing that sequence of DNA from the nucleic acid fragments of the library. The present method for producing a nucleic acid library for data storage may therefore comprise a further step v) of encoding digital data into individual oligonucleotide fragments. For the purpose of data storage, a sequence that follows an arbitrary coding scheme, for example 00=A, 01=G, 10=C, 11=T, to encode digital data can be produced by building a specific DNA sequence of "n" length and complexity. Another option is to assign two of the nucleic acid bases with a 0 and the other two nucleic acid bases with a 1. The coding scheme can further be elaborated to include tags and encryption codes. The coding scheme can also be modified to encompass different challenges specific to each fragment. For example, compressing algorithms may be applied, or simplified codes may be used such as binary codes utilizing only two nucleotides to encode the digital data, for example 0=G, 1=C while A and T are discarded.

By assigning each base with a binary code, each oligonucleotide fragment will represent a specific sequence of binary code. The oligonucleotide fragments in the library can then be combined in any way to form longer and more complex sequences to make composite fragments with a determined sequence at will. Utilizing this library, the generation of oligonucleotide sequences of different length and complexity for any application requiring specific DNA sequences will be possible. To form longer fragments, the oligonucleotide fragments are joined using any commonly used method for joining nucleic acids to each other, such as by using ligases or other enzymatic ligations or targeted biochemical processes like the CRISPR technology. The present method for producing a nucleic acid library for data storage may therefore comprise a further step vi) of assembling the individual oligonucleotide fragments into longer nucleic acid sequences, such as by ligation or CRISPR/Cas9 technology, for storage of data. For enzymatic assembly, the oligonucleotide fragments in the library can be adapted with overhangs or tags which enables controlled ligation of the oligonucleotide fragments. A ligase catalyzes the reaction between the phosphate group of the 5' end and the hydroxyl group off the 3' end. If blunt end fragments are ligated (without adaptive tags), the reaction is controlled in space and time to a) avoid inclusion of multiple oligonucleotide fragments and b) ensure ligation occurs in one direction only. This can be achieved by e.g., microfluidic control, attaching the growing fragment to a support, and/ or by usage of 5' and 3' modifications.

By preparing oligonucleotide fragments in the size ranges disclosed herein, it is possible to obtain a nucleic acid library having a sufficient size to enable the coding of virtually any code thereinto while still having a library that is of reasonable size. For example, if shorter oligonucleotide fragments are produced, the library will be of a smaller size but will also be more difficult to handle as the number of ligations to produce complex fragments will increase. On the other hand, if longer oligonucleotide fragments are produced the library size would have to be very large in order to maintain the flexibility in coding options. The size ranges disclosed herein are suitable for creating a nucleic acid library of reasonable size but that still allows virtually any code to be coded thereinto.

One advantage of the present method for producing a nucleic acid library for data storage is that longer nucleic acid sequences can be produced by assembling the shorter oligonucleotide fragments of the library into longer nucleic acid sequences. Thereby, nucleic acid sequences of any length may be constructed, such as nucleic acid sequences having at least 1000 bp, such as at least about 2000 bp, such as at least about 3000 bp, such as at least 4000 bp, such as at least 5000 bp, such as from about 3000 bp to about 100000 bp. Thereby, the problems with synthesising long enough nucleic acid fragments for data storage is avoided. Also, the size of the oligonucleotide fragments of the present nucleic acid library produced and the fact that they can be combined in any order and/or number allow a great flexibility in data storage for both simpler and more complex codes. Thereby, virtually any data code may be stored by assembling oligonucleotide fragments of the nucleic acid library into a longer nucleic acid sequence having the necessary sequence to encode the data in the correct way.

When longer nucleic acid sequences are constructed using the oligonucleotide fragments, problems with secondary structure formation may occur. These problems can be mitigated or prevented by chromatinizing the longer nucleic acid sequences. Such chromatinization may be performed by any available method for chromatinization. One example of such a method is provided in the Experimental section, but the present document is not limited to the use of this specific method for chromatinization. DNA may for example be chromatinized by incubation with histone octamers and histone chaperones followed by a stepwise salt gradient dialysis. The result of this process is the folding of the growing DNA strands in a stable conformation with the histone proteins ensuring the strand does not break, entangle, or form secondary structures which could be prohibiting to further processing of the nucleic acid sequences.

The nucleic acid may be any nucleic acid such as DNA, RNA or cDNA. The nucleic acid may be native or synthetic nucleic acid. However, the nucleic acid of step i) of the present method for producing a nucleic acid library for data storage is an existing nucleic acid, i.e. the present method utilizes a source of existing nucleic acid for preparing the nucleic acid library rather than *de novo* synthesizing nucleic acid to provide the individual fragments of nucleic acid of the nucleic acid library. Preferably, the nucleic acid of the library is in the form of DNA as DNA is very stable over time. Preferably, the nucleic acid is native nucleic acid as there are abundant sources of native nucleic acid available. If the nucleic acid used for preparing the nucleic acid library is RNA, preferably reverse transcribe the RNA to cDNA to increase stability of the oligonucleotide fragments. Also, it is possible to maintain and/or amplify the oligonucleotide fragments of nucleic acid using PCR/other amplification methods such as isothermal amplification e.g., NASBA amplification, MDA or LCA amplification as well as by novel generation of oligonucleotides from existing DNA. The oligonucleotide fragments of the library may therefore be oligonucleotide fragments of the nucleic acid used to prepare the library or oligonucleotide fragments generated based on the nucleic acid. It is important to note that also in the latter case, the nucleic acid of step i) of the present method is an existing nucleic acid from any source.

In the first step of the present method for producing a nucleic acid library for data storage, a nucleic acid is provided. If the nucleic acid is a native nucleic acid, typically, this step involves providing a native nucleic acid by extracting it from a nucleic acid containing biological material. The nucleic acid containing biological material is not critical but can be any nucleic acid containing material, such as organisms which can be cheaply cultured in the lab, like bacteria, such as *E. coli,* yeasts, plants or any other available source of nucleic acid such as disposed waste food. DNA and RNA extraction methods are common practice in any molecular biology laboratory and have been described and optimized over several decades. Protocols for DNA and RNA extraction are standard procedures and any protocol for DNA or RNA extraction can be used for this step. The step of extracting native nucleic acid may optionally be followed by a step of cleaning up the extracted native nucleic acid. Impurities remaining in isolated nucleic acid may be removed by any standard procedure to this end. Exemplary and non-limiting methods for cleaning up extracted DNA or RNA are given in the Experimental section. As mentioned above, instead of using native nucleic acid, it is possible to use existing synthetic nucleic acid from any source.

The second step of the present method for producing a nucleic acid library for data storage involves fragmenting the nucleic acid of step i) to provide oligonucleotide fragments of nucleic acid. The fragmentation results in random oligonucleotide fragments of the nucleic acid. DNA extraction typically results in varying oligonucleotide lengths ranging from various sizes at the kilobase scale. If RNA is used as the nucleic acid, fragments typically are shorter, around 2kb. However, the initial length of the extracted nucleic acid chains is not critical for the present method and no size selection is necessary prior to the fragmentation. The isolated nucleic acids can be fragmented by any method allowing fragmentation of nucleic acid. Typically, the nucleic acid is fragmented by enzymatic or physical (sonication, nebulization, different types of shearing or pressure cell passage) methods. Thereby thousands of oligonucleotide fragments of different sizes are produced in a controlled manner. A non-limiting exemplary method for nucleic acid fragmentation is given in the Experimental section.

The step of fragmenting the nucleic acid may be followed by a step of separating the oligonucleotide fragments of nucleic acid based on size. This size-based isolation may be performed by any suitable method for isolating nucleic acid based on size, such as by gel separation, size exclusion chromatography or ultrafiltration. A non-limiting and exemplary gel separation method is disclosed in the Experimental section.

The oligonucleotide fragments of nucleic acid produced according to the present document may be end repaired and/or tagged. Such end repair and/or tagging is preferably performed before or after step iii) and/or before or after step iv). A non-limiting and exemplary method for end repair producing blunt ends is disclosed in the Experimental section.

In the third step of the present method for producing a nucleic acid library for data storage, the oligonucleotide fragments produced are isolated. The aim of this step is to isolate the oligonucleotide fragments in a way so that individual oligonucleotide fragments are obtained. The isolation of oligonucleotide fragments may be achieved in several ways. For example, by serial dilutions or by utilizing microfluidic printing, a microfluidic droplet generator or microfluidic compartmentalization. Using a microfluidic separation will allow automatization of the process and allows us to separate single oligonucleotide fragments in microfluidic volumes or by using emulsion drops. Regardless of the method of dilutions, the principle remains valid. By serial dilutions of the sample to sufficiently small volumes, volumes containing (on average) one fragment of DNA were obtained. The dilutions produce some volumes or droplets containing nothing and other volumes or droplets containing one or several oligonucleotide fragments. By optimization, a protocol avoiding multiple oligonucleotide fragments per volume can be established, which allows for perfect separation of the oligonucleotide fragments. The volumes or droplets can be managed by different types of automatic separation such as microfluidic printing, compartmentalization or well deposition from microfluidic volumes. Other types of microfluidic automatizations for managing the dilutions may also be considered. According to the present document, isolation of the oligonucleotide fragments may thus e.g., be performed by serial dilution, microfluidic printing, microfluidic compartmentalization, and/or microfluidic droplet generation. In the Experimental section, a non-limiting example of oligonucleotide fragment isolation using serial dilution is given.

Step iv) is an optional step of the present method for producing a nucleic acid library for data storage, wherein the individual oligonucleotide fragments of nucleic acid obtained in step iii) are sequenced. The method used for such sequencing is not critical and any method allowing sequencing of DNA or RNA may be used. The sequencing of the individual oligonucleotide fragments may be performed in connection with producing the library so that the sequence of the individual oligonucleotide fragments of the library are known. Alternatively, it is possible to prepare a library of individual oligonucleotide fragments of unknown sequences and sequence the individual oligonucleotide fragments at a later time point.

The individual oligonucleotide fragments of known or unknown sequence are then archived for future usage in a nucleic acid library. To archive nucleic acid sequences for data storage in a library, tags barcodes or other position numbering may be used. To protect the oligonucleotide fragments from degradation, they may be embedded in different mineral coatings that protect the oligonucleotides against time but also against environmental insults. The present method for producing a nucleic acid library for data storage may therefore comprise a further step of embedding the oligonucleotide fragments (or longer nucleic acid sequences produced by assembling several oligonucleotide fragments into longer nucleic acid sequences) in a mineral residue or other preservation processes. As mentioned above, longer nucleic acid sequences constructed by assembling oligonucleotide fragments may also be chromatinized to mitigate or prevent problems with secondary structure formation.

The present document is also directed to a nucleic acid library for data storage, wherein the nucleic acid library for data storage is obtained or obtainable by the method for producing a nucleic acid library for data storage disclosed herein.

The present document is also directed to a method for storing data, which method comprises storing data using the herein described nucleic acid library for data storage. The method for storing data may comprise assembling the individual oligonucleotide fragments of the nucleic acid library disclosed herein into longer nucleic acid sequences, such as by ligation or utilizing CRISPR/Cas9 technology, to obtain a nucleic acid sequence with a sequence corresponding to the data code it is to encode. The longer nucleic acid sequences may optionally be chromatinized and/or embedded in a mineral coat as disclosed elsewhere herein.

The present document is also directed to the use of nucleic acid for the preparation of a nucleic acid library for data storage.

The invention will be further described in the following examples, which do not limit the scope of the invention described in the claims.

### EXPERIMENTAL SECTION

### Materials and Methods:

### DNA and RNA extraction:

DNA and/or RNA was extracted from any living organism or any biological material containing DNA and RNA using published and well-established DNA/RNA extraction protocols. DNA and RNA extraction methods are common practice in any molecular biology laboratory and have been described and optimized over several decades.

Protocols for DNA and RNA extraction are standard procedures and any protocol for DNA or RNA extraction can be used for this step.

### Measurement of concentration of nucleic acids and fragment size:

Measurements were made by usage of Nanodrop spectrophotometer according to the manufacturer's instructions. Qubit^{™} fluorometer and Aglient 2100 Bioanalyzer instrument were used to determine the concentration and size distribution of the DNA samples, also according to the manufacturer's instructions. Qubit^{™} 1X dsDNA High Sensitivity (HS) Assay Kit or QubitTM dsDNA Broad Range (BR) Assay Kit and Assay tubes (Invitrogen, Thermo Fisher Scientific) and the Agilent DNA 1000 Kit (Agilent Technologies) were used.

All measurements were done in duplicates, and the average value was reported.

### Clean up of isolated nucleic acids:

Impurities remaining in the isolated DNA were removed by adding a step of extra centrifugation at 13,000 × g for 10 min after the DNA isolation. The supernatant was recovered after centrifugation and further cleaned by usage of AMPure beads (Beckman Coulter^{™}) according to manufacturer's instructions. AMPure beads were added to the DNA samples at a volume of 1.8X, the solution was mixed and incubated for 10 min at room temperature. Beads were attached to magnets and the liquid removed. Beads (still attached to magnets) were rinsed twice with 300 µL 70% ethanol. Ethanol was removed and the samples were left to dry for 10-15 min. Samples were then removed from the magnets, 1X Tris buffer was added, solution was mixed thoroughly, incubated for 7 min at room temperature and transferred to new 1.5 ml tubes (while beads were bound to the magnets).

When RNA is used as a starting source for nucleic acids, RNA can be cleaned up by usage of commercially available spin columns.

When RNA is used for the experiments, the RNA is reverse transcribed to cDNA to increase stability of the oligonucleotides. For this purpose, QuantiTect Reverse transcription Kit (Qiagen) for cDNA preparation may be used, but any kit available on the market can be used. To prepare the mastermix 1 µg of RNA is mixed with 2 µL gDNA wipeout buffer and H₂O to a total volume of 14 µL. The reaction is incubated for 2 min at 42°C, then placed on ice. In parallel, a second mastermix of 1 µL Quantiscript reverse transcriptase, 4 µL RT Buffer, and 1 µL RT Primer mix is prepared, 6 uL of mastermix 2 is added to the first reaction which is first incubated for 15 more min at 42°C and then 3 min at 95°C to inactivate the Quantiscript Reverse Transcriptase.

### Fragmentation of nucleic acids:

The present document and method are based on the usage of existing strands of DNA or RNA to generate random oligonucleotide fragments. DNA extraction typically results in varying oligonucleotide lengths ranging from various sizes at the kilobase scale. If RNA is used fragments are shorter, typically around 2kb, the initial length of the nucleotide chains (the starting material) does not impact the results of these experiments, and no size selection is necessary prior to the fragmentation.

The isolated nucleic acids can be fragmented by different methods; enzymatic or physical (sonication, nebulization, different types of shearing or pressure cell passage) to produce thousands of oligo-sequences in a controlled manner. Here Micrococcal Nuclease 2,000,000 units/ml (NEB) was used according to the manufacturer's instructions (but any other Nuclease that fragments DNA randomly can also be used for the reaction). The enzymatic reactions were performed on ice. Micrococcal Nuclease was diluted to the desired concentration with DNase/RNase-Free distilled water (Invitrogen). A mastermix was prepared by mixing 5 µl Micrococcal Nuclease Reaction Buffer 10X, 0.25 µl recombinant albumin (20 mg/mL), 0.75 µl Micrococcal Nuclease, 10 µg of DNA, and H₂O to a total volume of 50 µl. The solution was mixed and incubated for 2 min at 37°C. 2 µl of EGTA (500mM, pH 8.0) was added to stop the reaction, followed by mixing and a second incubation at 62°C, for 10 minutes.

### Gel electrophoresis

2% agarose gels were prepared by mixing agarose (ThermoScientific) with TAE buffer, microwaving the solution, and pouring it into a cast with combs. 10 µL of the DNA samples were mixed with 2 µL loading dye (NEB) and loaded on the gel. TriDye ultra-low range DNA ladder (NEB) and Generuler 1 kb plus DNA ladder (Thermo Scientific) for size reference were used. Electrophoresis was run in 0.5X TAE buffer at 70V for at least 1h to allow separation of smaller DNA fragments. Gel staining was performed after electrophoresis by placing the gels for 30 min in a solution with 1X SybrSafe (Invitrogen) in 0.5X TAE buffer.

### Isolation of DNA from gel:

This protocol was maintained from a previous publication by Obrador-Sánchez *et al.* 2017.

Tubes for DNA extraction were prepared as described by Obrador-Sánchez *et al.* Small pieces of cotton were soaked in 0.5X TAE buffer and placed at the bottom of the 0.6 mL microcentrifuge tube which had been pierced in the bottom with a needle. The cotton should occupy approximately one third of the volume of the tube. Pressure was applied to the cotton until it was not possible to extract anymore buffer. The 0.6 mL microcentrifuge tubes with cotton were then placed on top of 1.7mL low binding tubes.

In parallel, gels were placed on a UV table to visualize the DNA, bands of interest were identified and cut out with a scalpel. The pieces of gel were placed in the tubes with cotton and centrifuged as described by Obrador-Sánchez et al. If needed, centrifugation time can be increased to improve DNA recovery.

### Fragment end repairs and tagging:

Fragment end repairs with the aim of achieving only fragments with blunt ends was done by usage of Polymerase I Large (Klenow) (NEB). 1 µg of DNA was mixed with 4.5 µl of NEBuffer^{™} 2 buffer (10X), 1.5 µl of each dNTP (10mM) and Ultra-pure H₂O (Invitrogen) to a total volume of 45 µL. Reaction was mixed by vortexing, 1 unit of DNA Polymerase I, Large (Klenow) was added, and the reaction was incubated for 15 minutes at 25°C, at 300 rpm. The reaction was stopped by adding EDTA to a final concentration of 10 mM and heating for 20 minutes at 75°C. Herein repair of overhangs was used, but fragments can also be tagged by short oligos or by modified nucleotides for example using biotinylated or fluorescein labeled nucleotides to generate differently tagged fragments for the library.

### Fragment isolation:

The isolation of fragments may be achieved in several ways. For example, by serial dilutions or by utilizing microfluidic separation such as microfluidic printing, microfluidic compartmentalization, or a microfluidic droplet generator. Using a microfluidic separation will allow automatization of the process and allows us to separate single fragments in microfluidic volumes or by using emulsion drops. Regardless of the method of dilutions, the principle remains valid. By serial dilutions of the sample to sufficiently small volumes, volumes containing (on average) one fragment of DNA were obtained. The dilutions produce some volumes or droplets containing nothing and other volumes or droplets containing one or several oligo fragments. By optimization, a protocol avoiding multiple fragments per volume can be established, which allows for perfect separation of the fragments. The volumes or droplets can be managed by different types of automatic separation such as microfluidic printing, compartmentalization or well deposition from microfluidic volumes. Other types of microfluidic automatizations for managing the dilutions may also be considered.

Below is a description of how fragments were isolated by serial dilutions. The relation between molarity, concentration and fragment size was calculated manually using the equation: Molarity (nM/L) = (concentration (ng/µL) / (size of fragment in bp * average molecular weight of a base pair (g/mol))) * 10^6

The estimated average molecular weight of a base pair was 654 g/mol.

To calculate the number of molecules per µL based on the DNA concentration (in ng/µL) and the length of the DNA molecules (in bp) Avogadro's Number = 6.022 × 10^23 molecules/mol and the average molecular weight of a base pair were used. The number of molecules of DNA in each sample = amount of dsDNA x Avagadro's Number In this example the Number of molecules in 1 µL = Avogadro's Number * Concentration (ng/µL) / (number of bp*molecular weight* 10^9).

### Post processing of fragments:

Characterization of the produced oligos is performed by sequencing utilizing commercially available tools. The sequences are then archived for future usage. To archive sequences for data storage in a library, tags barcodes or other position numbering are used. The pool of oligos can be maintained and amplified using PCR/other amplification methods such as isothermal amplification e.g., NASBA amplification, MDA or LCA amplification as well as by novel generation of oligonucleotides from existing DNA.

For long term storage (centuries to millennia) of sequences generated they can be embedded in different mineral coatings that protect the oligonucleotides against environmental insults and enzymes that break DNA.

### Fragment assemblies:

To create longer complex fragments with a determined sequence enzymatic assemblies are performed by means of ligations and/or CRISPR technology. For enzymatic assemblies, the fragments in the library can be adapted with overhangs or tags which enables controlled ligation of the fragments. A ligase catalyzes the reaction between the phosphate group off the 5' end and the hydroxyl group off the 3' end. If blunt end fragments are ligated (without adaptive tags), the reaction is controlled in space and time to a) avoid inclusion of multiple fragments and b) ensure ligation occurs in one direction only. This can be achieved by microfluidic control, attaching the growing fragment to a support, and/ or by usage of 5' and 3' modifications.

### Generation of chromatinized DNA:

Purified DNA is diluted to ~100-500 ng/µL in TE buffer (10 mM Tris, 1 mM EDTA, pH 7.5). Histone octamers are prepared as recommended by manufacturer.

A Salt Gradient Nucleosome Assembly is prepared by mixing DNA and Histone octamers in a molar ratio of 1:1.2 (DNA to histones) in an assembly buffer containing 2 M NaCl in a total reaction volume of 20-50 µL. After mixing, the reaction is incubated at high salt concentration for 15 minutes at room temperature to allow histone octamers to interact with DNA. Transfer the DNA-histone mixture to a dialysis tube or micro-dialysis button. Perform stepwise salt gradient dialysis by gradually reducing the NaCl concentration (start with 2 M NaCl and reduce stepwise to 1.5 M, 1 M, 0.5 M and last 0.2 M NaCl). Hold each step for 30 minutes to 1 hour at 4°C with gentle agitation.

For improved assembly, histone chaperones can be included during the assembly process.

The chromatinized DNA can be stored at 4°C for short-term use or -80°C for long-term storage. Repeated freeze-thaw cycles are to be avoided.

### Results:

For the purpose of data storage, a sequence that follows an arbitrary coding scheme, for example 00=A, 01=G, 10=C, 11=T, to encode digital data can be produced by building a specific DNA sequence of "n" length and complexity. The coding scheme can further be elaborated to include tags and encryption codes. The coding scheme can also be modified to encompass different challenges specific to each fragment. For example, compressing algorithms may be applied, or simplified codes may be used such as binary codes utilizing only two nucleotides to encode the digital data, for example 0=G, 1=C while A and T are discarded. Once translated, a nucleotide sequence of a predetermined order needs to be produced to contain the information. Herein it is proposed to enable DNA data storage by developing an entirely novel DNA synthesis platform which does not depend on complex chemistries or controlled enzymatic reactions. This new method of DNA synthesis is based on usage of existing DNA or RNA. Any source of DNA or RNA can be utilized as a starting material, i.e., any biological organism containing DNA or RNA, e.g., bacteria, archaea, and eukaryotes, but also any disposed organic material containing DNA or RNA (like food waste) to produce nucleic acids using standard protocols.

Herein frozen corn purchased from the grocery store was used to extract DNA to demonstrate the method.

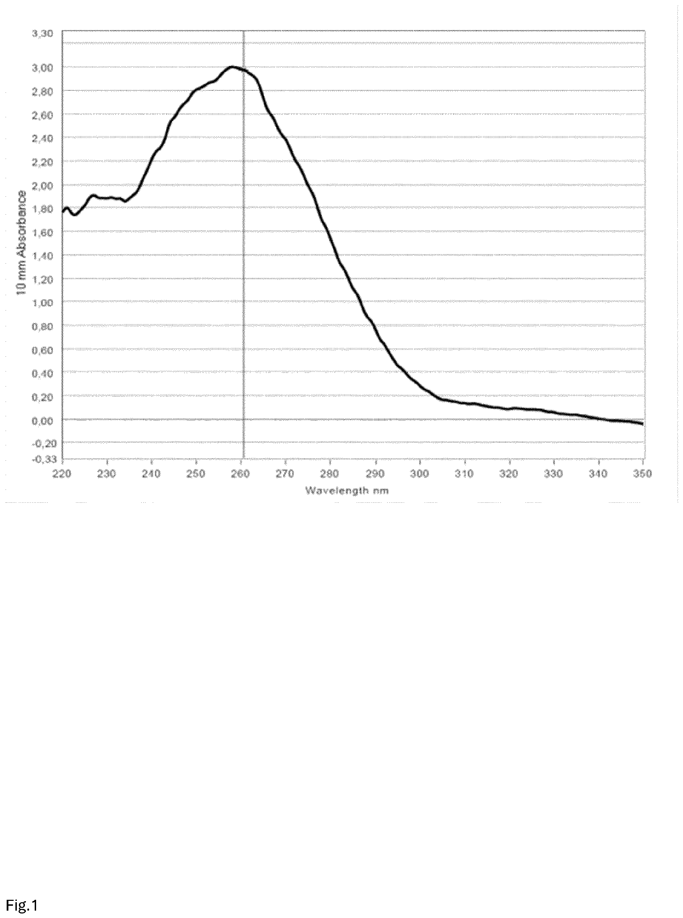

DNA isolation from biological sources may yield impurities like proteins or polysaccharides, which can be removed using proteinase K or DNA-binding beads. Here, from four samples an average 260/280nm measurement of 1.72 and an average 260/230nm measurement of 0,80 was achieved. Polysaccharide leftovers were eliminated by including additional steps of centrifugation in the last steps of the DNA isolation protocol, and by usage of AMPure beads on the isolated DNA. Measurement on the four pooled samples is shown in Figure 1, displaying a sufficient purity for downstream applications.

By fragmentation, random chains of oligonucleotides were generated to build a massive library of short oligo sequences. The oligonucleotides are cataloged and archived in an oligonucleotide library for use in the generation of longer sequences of DNA in a later step. Here, DNA isolated from corn was conveniently fragmented by usage of Micrococcal Nuclease restriction enzyme, but other methods (physical fragmentation) may also be applied. High concentrations of the nuclease and prolonged reaction times resulted in total digestion of the DNA (Figure 2A). The protocol was adapted to achieve a mean fragment size of 10-100 base pairs. The results indicated that dilution of the Micrococcal Nuclease to 1:8 or 1:16 was suitable in this case, resulting in a fragmentation of the DNA to small oligonucleotides, with a peak of a size smaller than 15 bp when diluting 1:8 and a peak at 15 when diluting 1:16 (Figures 2-3). Dilution of the Micrococcal Nuclease to 1:32 and 1:64 generated larger fragments of 50 base pairs or more.

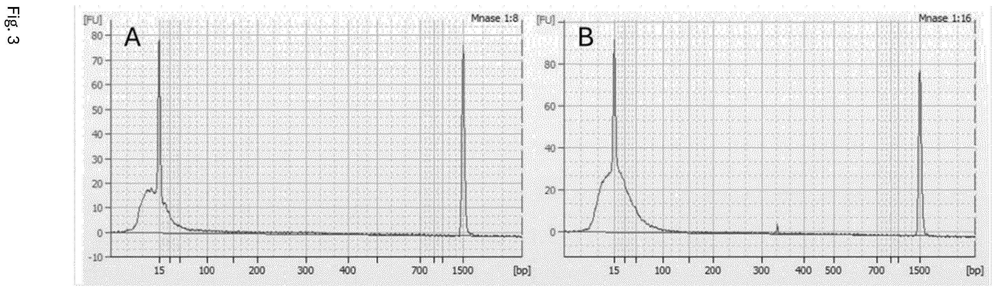

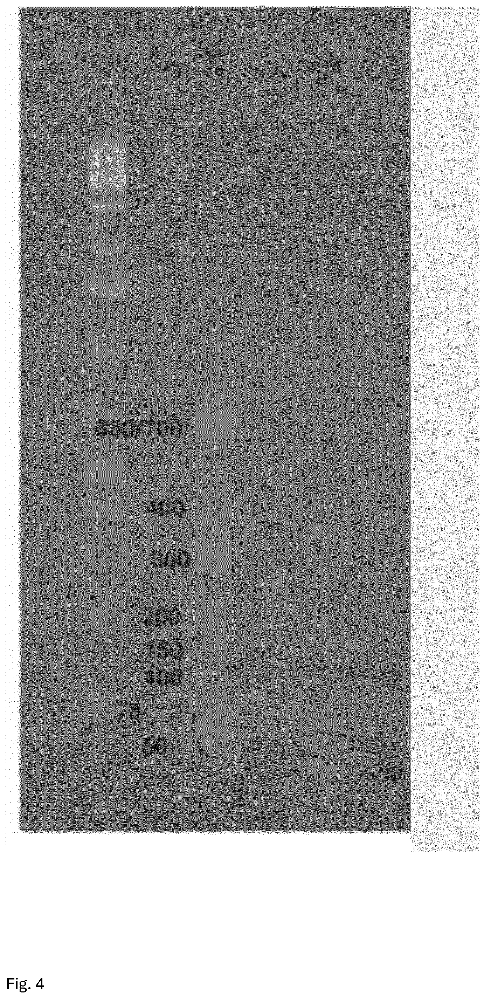

The estimated size of the oligonucleotides obtained from fragmentation was further confirmed by isolation of fragments from a 2% agarose gel. Digested DNA can be seen as a diffuse smear on the bottom part of the gel corresponding to oligonucleotides of smaller size. Gel fragments corresponding to bands of an estimated size of 100, 50 and below 50 base pairs (marked by circles on the gel in Figure 4) were cut out, and the DNA isolated from the gel-pieces. DNA concentrations from the larger range of oligonucleotides were below the range of detection as measured with Qubit^{™} (dsDNA High Sensitivity Assay Kit), which confirms the estimated size distribution from the bioanalyzer electropherograms in Figure 3.

The isolation of fragments from an agarose gel is optional and can be omitted from the protocol. It was included here for demonstration of the fragmentation protocol and for narrowing down the range of sizes of fragments, which may not be necessary depending on the dilution of enzymes used and the requirement of the fragments that are produced. It should also be noted that extraction of DNA fragments from gel reintroduced some impurities to the samples, generating low 260/280 and 260/230 reads (Table 1), these impurities may have to be removed depending on downstream applications, this aspect should also be considered before including this step in the protocol.

**Table 1: DNA concentration and quality after size selection and DNA extraction from agarose gels. *Note that Qubit measurements, which are the most precise at low DNA concentrations, showed no presence of double stranded DNA fragments in the sample from the 100 bp gel extraction (LOD = Limit of detection). Extraction of DNA fragments from gel resulted in some impurities in the sample as can be seen by the 260/280 and 260/230 readouts.**

| Targeted size | C ng/µL (NanoDrop) | 260/280 nm | 260/230 nm | Qubit HS ng/µL |
|---|---|---|---|---|
| 100 bp | 16.9 | 1.15 | 0.64 | <LOD* |
| 50 bp | 19.9 | 1.13 | 0.73 | 0.110 |
| < 50 bp | 16.4 | 1.32 | 0.61 | 0.073 |

Micrococcal Nuclease randomly digests DNA and produces double stranded DNA fragments. Around 40% of the fragments have blunt ends and the rest have short overhangs of a few base pairs (Harkins *et al.* 2020). Overhangs can be repaired if on the 5' strand or blunted if on the 3' strand by usage of T4 DNA Polymerase or DNA Polymerase I enzymes. Here, DNA Polymerase was used to repair ends of the fragments and generate DNA fragments with 100% blunt ends for downstream applications.

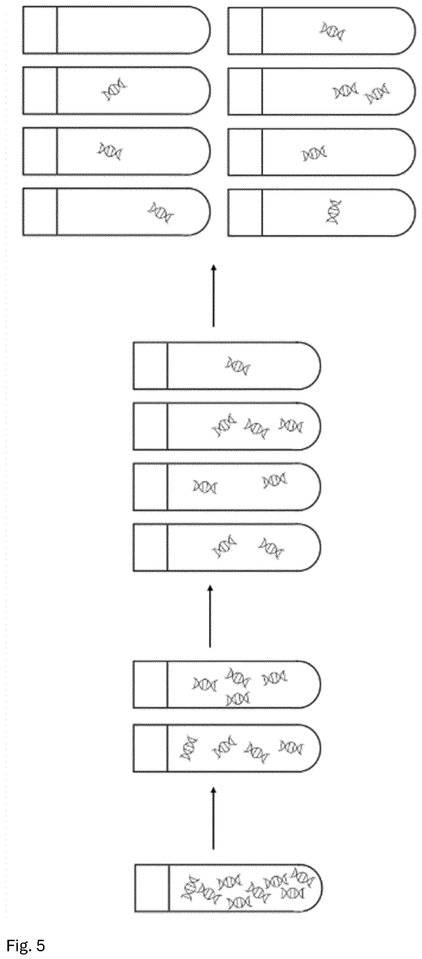

After fragmentation, the oligonucleotides generated by fragmentation of DNA to form the basis of the library were isolated. Based on the principle that a perfect dilution of fragments will enable their separation we achieved single fragment separation by diluting and splitting the volume containing the fragments to sufficiently small volumes. When the dilutions reach sufficiently low concentrations, in theory each volume contains one single fragment of DNA (Figure 5). In practice, some volumes will contain more than one fragment, and some volumes will contain no fragments, these volumes will be discarded at a later stage.

The average length of DNA fragments in the sample was estimated based on the concentration and molarity measurements obtained using Aglient Bioanalyzer by usage of the formula Molarity (nM/L) = (concentration (ng/µL) / (size of fragment in bp * average molecular weight of a base pair (g/mol))) * 10^6, the average molecular weight of a base pair (double stranded) was estimated to 654 g/mol. In Table 2 are displayed the Agilent 2100 Bioanalyzer measurements made in the samples and the calculated fragment sizes. Fragments of a size roughly corresponding to the expected size based on the size selection from agarose gel cuttings were observed. Only one sample (1:32 cut at 100 bp) was not in the expected range.

**Table 2: Estimated length of fragments isolated from gel.**

| Digestion | Gel cut out | C (ng/ µL) | Molarity (nmol/l) | Estimated length (bp) |
|---|---|---|---|---|
| 1:16 | <50 | 1.99 | 59.8 | 47.54 |
| 1:16 | 50 | 1.48 | 47.8 | 51.23 |
| 1:16 | 100 | 0.98 | 21.3 | 70.77 |
| 1:32 | <50 | 0.83 | 51.7 | 24.77 |
| 1:32 | 50 | 4.01 | 116.4 | 52.92 |
| 1:32 | 100 | 1.59 | 86.5 | 28.31 |

Based on the assumption that all fragments in one sample are of approximately the same size these measurements can be used to calculate dilution series for the separation of DNA to volumes containing 1 single fragment as illustrated in Figure 5.

To calculate the number of DNA molecules per µL based on the DNA concentration (in ng/µL) and the length of the DNA molecules (in bp) the following equation was used: The number of molecules of DNA in each sample = amount of dsDNA x Avagadro's Number. Avogadro's Number = 6.022 × 10^23 molecules/mol. In this example the Number of molecules in 1 µL = Avogadro's Number * Concentration (ng/µL) / (number of bp*molecular weight * 10^9)

Using the first sample in Table 1 is used as an example the following is calculated: $Number of molecules in 1 μL = 6.022 \times {10}^{∧} 23 * 1.99 / \left(47.54*645*{10}^{∧}9\right)$ $Number of molecules in 1 μL = 3.91 \times {10}^{∧} 10$

This volume can be diluted to 10^10 by a dilution factor of 3.91 (mix 3.91 µL of DNA with 35.09 uL H₂O or buffer). This volume can further be diluted by a 10-fold dilution series (8 times) to a theoretical concentration of 100 molecules in a volume of 1 mL and then distributed in volumes of 10 µL in a 96 well plate to achieve a final concentration of one molecule DNA in each well containing 10 µL solution.

As demonstrated here, fragments were isolated by principles of serial dilution. But the isolation of fragments may also be achieved in other way, for example, by utilizing a microfluidic separation or droplet generator, single fragments can be separated in microfluidic volumes or emulsion drops.

These fragments are the building blocks and form a library of sequences that can be used for any secondary purpose, by themselves or in different combinations. The fragments of different lengths build a vast library of "n" DNA and RNA sequences which can be elastic in fragment sizes and random. The produced and fragmented DNA and RNA can be tagged and postprocessed before or after the separation. Herein the repair of overhangs prior to separation is described, but fragments can also be tagged by short oligos or by modified nucleotides for example using biotinylated nucleotides to generate differently labeled fragments for the library.

Fragments in the library can be combined in any way to form longer and more complex sequences to make composite fragments with a determined sequence at will. Utilizing this library, the generation of oligonucleotide sequences of different length and complexity for any application requiring specific DNA sequences will be possible. To form longer fragments, the joining of different oligo sequences may be catalyzed by ligases or other enzymatic ligations or targeted biochemical processes like the CRISPR technology.

In order to increase the stability of DNA fragments and to generate longer synthetic DNA fragments, molecular biology strategies can be utilized. Specifically, by the generation of chromatinized DNA and usage of the condensin complex DNA fragments can be stabilized permitting the elongation of fragments beyond the current commercial limits of 5000 bp. DNA is chromatinized by incubation with histone octamers and histone chaperones followed by a stepwise salt gradient dialysis. The result of this process is the folding of the growing DNA strands in a stable conformation with the histone proteins ensuring the strand does not break, entangle, or form secondary structures which could be prohibiting to further processing of the fragments.

### Discussion:

The present document presents a new methodology for digital data storage in nucleic acid, preferably DNA. Compared to other solutions, the solution presented in the present document is unique in the combination of fragmentation of existing, preferably native, nucleic acid sequences and generating new combinations of said sequences. In this document are described the crucial steps of the technology, from the isolation of DNA from organic material to the cleanup, fragmentation, repair, and isolation of the new sequences that are incorporated into a dynamic oligonucleotide library which will be the basis of synthesis of new DNA strands.

In contrast to prior art technologies which rely on synthetic fragments to build their library, which makes the solution dependent on expensive base-by-base synthesis of DNA, the present method relies on the generation of new library fragments from existing biological material such as waste. This is a more efficient solution in many aspects and implies lower costs and less resources used.

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

Unless expressly described to the contrary, each of the preferred features described herein can be used in combination with any and all of the other herein described preferred features.

### REFERENCES

WO 2016/059610
WO 2021/231920
US11538554
US2020063119
Harkins Kelly M., Schaefer Nathan K., Troll Christopher J., Rao Varsha, Kapp Joshua, Naughton Colin, Shapiro Beth, and Green Richard E. 'A Novel NGS Library Preparation Method to Characterize Native Termini of Fragmented DNA'. Nucleic Acids Research 48, no. 8 (7 May 2020): e47. https://doi.org/10.1093/nar/gkaa128
Obrador-Sánchez A.J., Tzec-Sima M., Higuera-Ciapara I., and Canto-Canché B.. 'A Quick and Effective In-House Method of DNA Purification from Agarose Gel, Suitable for Sequencing'. 3 Biotech 7, no. 3, July 2017: 180. https://doi.org/10.1007/s13205-017-0851-1.
Sandoval-Velasco M., Dudchenko O., Rodriguez J.A., Pérez Estrada C., Dehasque M., Fontsere C., Mak S.S.T., Khan R., Contessoto V.G., Oliveira A.B.Junior, Kalluchi A., Zubillaga Herrera B.J., Jeong J., Roy R.P., Christopher I., Weisz D., Omer A.D., Batra S.S., Shamim M.S., Durand N.C., O'Connell B., Roca A.L. Plikus M.V., Kusliy M.A., Romanenko S.A., Lemskaya N.A., Serdyukova N.A., Modina S.A., Perelman P.L., Kizilova E.A., Baiborodin S.I., Rubtsov N.B., Machol G., Rath K., Mahajan R., Kaur P., Gnirke A., Garcia-Trevino I., Coke R., Flanagan J.P., Pletch K., Ruiz-Herrera Az., Plotnikov V., Pavlov I.S., Pavlova N.I., Protopopov A.V., Di Pierro M., Graphodatsky A.S., Lander E.S., Rowley M.J, Wolynes P.G., Onuchic J.N., Dalen L., Marti-Renom M.A., Gilbert M.T.P., and Lieberman A.E. 'Three-dimensional genome architecture persists in a 52,000-year-old woolly mammoth skin sample'. Cell, Volume 187, Issue 14, 3541 - 3562.e51, 2024. https://doi.orq/10.1016/j.cell.2024.06.002

## Claims

1. A method for producing a nucleic acid library for data storage, said method comprising the steps of:
i) providing a nucleic acid;
ii) fragmenting the nucleic acid of step i) to provide oligonucleotide fragments of nucleic acid;
iii) isolating the oligonucleotide fragments of nucleic acid obtained in step ii) to provide individual oligonucleotide fragments of nucleic acid;
iv) optionally sequencing the individual oligonucleotide fragments of nucleic acid obtained in step iii);
thereby providing a library of individual oligonucleotide fragments for data storage.

2. The method of claim 1, wherein the oligonucleotide fragments are from about 4 base pairs to about 30 base pairs long, such as from about 4 base pairs to about 25 base pairs, such as from about 4 base pairs to about 20 base pairs.

3. The method of any one of the preceding claims, wherein said method comprises a further step v) of encoding digital data into said individual oligonucleotide fragments.

4. The method of any one of the preceding claims, wherein step i) is performed by extracting native nucleic acid from a nucleic acid containing biological material, optionally wherein said step of extracting native nucleic acid is followed by a step of cleaning up the extracted native nucleic acid.

5. The method of any one of the preceding claims, wherein step iii) is performed by serial dilution, microfluidic printing, microfluidic compartmentalization and/or microfluidic droplet generation.

6. The method of any one of the preceding claims, wherein the individual oligonucleotide fragments of nucleic acid or longer nucleic acid sequences are embedded in a mineral residue.

7. A nucleic acid library for data storage, said nucleic acid library for data storage being obtained or obtainable by the method of any one of claims 1-6.

8. A method for storing data, said method comprising storing data using a nucleic acid library for data storage as defined in claim 7.

9. The method for storing data according to claim 8, said method comprising assembling individual oligonucleotide fragments of the nucleic acid library of claim 7 into longer nucleic acid sequences, such as by ligation or CRISPR/Cas9 technology, wherein said longer nucleic acid sequences optionally are chromatinized.

10. Use of nucleic acid for the preparation of a nucleic acid library for data storage.
